(19) Europäisches Patentamt
European Patent Office
Office européen des brevets

(11) **EP 2 565 200 B1**

(12) **EUROPEAN PATENT SPECIFICATION**

(45) Date of publication and mention
of the grant of the patent:
**11.10.2017 Bulletin 2017/41**

(51) Int Cl.:
*A23L 33/13* (2016.01)    *C09K 3/00* (2006.01)
*C07K 16/16* (2006.01)    *A61K 8/64* (2006.01)
*C07K 4/10* (2006.01)

(21) Application number: **11775158.6**

(22) Date of filing: **28.04.2011**

(86) International application number:
**PCT/JP2011/060485**

(87) International publication number:
**WO 2011/136377 (03.11.2011 Gazette 2011/44)**

(54) **ANTIFREEZE PROTEIN**

FROSTSCHUTZ-PROTEIN

PROTÉINE ANTIGEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.04.2010 JP 2010105847
30.04.2010 JP 2010105846**

(43) Date of publication of application:
**06.03.2013 Bulletin 2013/10**

(73) Proprietor: **KANEKA CORPORATION
Osaka (JP)**

(72) Inventors:
• **FUKUOKA, Joichi
Himeji-shi
Hyogo 679-2155 (JP)**
• **KAWAHARA, Hidehisa
Suita-shi
Osaka 564-8680 (JP)**
• **KEGASA, Hideaki
Takasago-shi
Hyogo 676-8688 (JP)**
• **ARAI, Naoki
Takasago-shi
Hyogo 676-8688 (JP)**
• **TOMONO, Jun
Takasago-shi
Hyogo 676-8688 (JP)**
• **YOKOTA, Shinichi
Takasago-shi
Hyogo 676-8688 (JP)**

(74) Representative: **Hoffmann Eitle
Patent- und Rechtsanwälte PartmbB
Arabellastraße 30
81925 München (DE)**

(56) References cited:
JP-A- 2001 245 659    JP-A- 2004 024 237
JP-A- 2004 275 008    JP-A- 2007 153 834
JP-A- 2007 169 246    JP-A- 2008 120 745
JP-A- 2009 131 246

• MAIKO FUKUURA ET AL.: 'Shushu no Seibutsushu Yurai no Futo Tanpakushitsu no Kassei Hyoka to Shokuhin Sozai ni Taisuru Kino Kaiseki' JAPAN SOCIETY FOR FOOD ENGINEERING DAI 8 KAI (2007 NENDO) NENJI TAIKAI KOEN YOSHISHU vol. 8, 2007, page 101, XP008162297
• HIDEHISA KAWAHARA ET AL.: 'Kaiware Daikon Yurai Futo Tanpakushitsu no Sonzai to Kino Kaiseki' NIPPON NOGEI KAGAKUKAI TAIKAI KOEN YOSHISHU vol. 2006, 2006, page 62, XP008162296
• HIDEHISA KAWAHARA ET AL.: 'Application in Food Industry and Function of Ice Crystal-controlling Materials Originated from Organisms' THE JOURNAL OF JAPANESE SOCIETY FOR CRYOBIOLOGY AND CRYOTECHNOLOGY vol. 55, no. 1/2, 2009, pages 49 - 53, XP008162298
• HIDEHISA KAWAHARA ET AL.: 'ANTIFREEZE ACTIVITY OF COLD ACCLIMATED JAPANESE RADISH AND PURIFICATION OF ANTIFREEZE PEPTIDE' CRYOLETTERS vol. 30, no. 2, 2009, pages 119 - 131, XP008162292

**Description**

TECHNICAL FIELD

The present invention relates to an antifreeze protein; a polypeptide which corresponds to an active part of the antifreeze protein; a composition, a food, a biological sample protectant and a cosmetic which respectively contain the antifreeze protein or the polypeptide; and an antibody which specifically responds with the antifreeze protein or the polypeptide.

BACKGROUND ART

An antifreeze protein is also referred to as a non-freezing protein or AFP. A general antifreeze protein shows effects such as inhibition of ice crystallization and control of ice crystal form, and also has a thermal hysteresis activity. Herein, thermal hysteresis means a temperature range which is less than the equilibrium melting point of the aqueous protein solution but in which ice cannot grow. The thermal hysteresis is detected as a difference between the equilibrium melting point and a freezing point when the freezing point is defined as a temperature at which ice starts to grow in the aqueous solution.

An antifreeze protein adsorbs on a surface of ice crystal to exhibit the above-described actions, and is utilized in the organisms for protecting the cells thereof from freezing. Such an antifreeze protein is found, for example, in a fish, an insect, a plant, fungi, a microorganism and the like (Patent Documents 1 to 2, Non-Patent Documents 1 to 4).

However, the antifreeze proteins reported so far are contained only in a very small amount in the living body of a fish, a plant, an insect, fungi, a bacterium or the like; therefore, extraction efficiency is very poor. Even if the antifreeze protein is present in a large amount, there is a problem that harvest or culture of the organism itself is difficult. Thus, the conventional antifreeze proteins cannot be industrially produced and utilized for food application.

Therefore, as a method for providing an antifreeze protein having an antifreezing activity stably throughout a year at low cost, a method of extracting an antifreeze protein using water or the like from a Japanese radish sprout preserved at low temperature is suggested (Patent Document 3). However, the antifreeze protein obtained by the method described in Patent Document 3 does not necessarily have enough antifreezing activity.

PRIOR ART DOCUMENT

PATENT DOCUMENTS

Patent Document 1: JP2004-24237A

Patent Document 2: JP2004-275008A

Patent Document 3: JP2007-153834A

NON-PATENT DOCUMENTS

Non-Patent Document 1: Biophysics, Vol.43, No.3, pp.130-135 (2003)
Non-Patent Document 2: Plant Physiology, Vol.119, pp.1361-1369 (1999)
Non-Patent Document 3: Biochem. J., Vol.340, pp.385-391 (1999)
Non-Patent Document 4: Can. J. Microbiol., Vol.144, p.6 (1998)

SUMMARY OF THE INVENTION

PROBLEMS TO BE SOLVED BY THE INVENTION

An objective of the present invention is to provide an antifreeze protein which is capable of being efficiently produced on an industrial level at low cost and which is safe and has an excellent antifreezing activity for use on a practical level. Also, an objective of the present invention is to provide a polypeptide that corresponds to the active part of the antifreeze protein; a composition, a food, a biological sample protectant and a cosmetic containing the antifreeze protein or the polypeptide; and an antibody that specifically reacts with the antifreeze protein or the polypeptide.

SOLUTIONS TO THE PROBLEMS

The present inventors intensively studied so as to solve the above problems. As a result, the inventors found a protein which has a very excellent antifreezing activity from a plant, to complete the present invention.

A first antifreeze protein (which does not form part of the present invention) is characterized in being derived from a plant, and comprising at least one of the following proteins:

(1) a protein having the amino acid sequence of SEQ ID NO:1 and having a molecular weight of 19, 000 ± 200 Da as measured by SDS-PAGE;
(2) a protein having the amino acid sequence of SEQ ID NO:2 and having a molecular weight of 22, 000 ± 250 Da as measured by SDS-PAGE;
(3) a protein having a molecular weight of 59, 000 ± 600 Da as measured by SDS-PAGE and having a modified N-terminal;
(4) a protein having the amino acid sequence of any one of the proteins (1) to (3) with one or more amino acid deletions, substitutions or additions, and having an antifreezing activity.

The second antifreeze protein according to the present invention is an antifreeze protein, being a plant seed storage protein, comprising at least one of the following amino acid sequences (1) to (5) :

(1) an amino acid sequence of SEQ ID NO: 3;
(2) an amino acid sequence of SEQ ID NO: 4;
(3) an amino acid sequence of SEQ ID NO: 5;
(4) an amino acid sequence of SEQ ID NO: 6;
(5) an amino acid sequence corresponding to any one of the amino acid sequence (1) to (4) with not less than 1 and not more than 5 of amino acid deletions, substitutions or additions, and having an antifreezing activity, wherein the antifreeze protein is obtainable from Japanese radish sprout, and wherein said protein is composed of two or more subunits, wherein a molecular weight of at least one subunit is 9000 Da ± 100 Da or 4000 Da ± 50 Da as measured by SDS-PAGE.

The polypeptide according to the present invention is characterized in being obtained by dissociating the above-described antifreeze protein according to the present invention, and having an antifreezing activity.

The antibody used in the present invention is characterized in specifically responding with the above antifreeze protein according to the present invention and/or the above polypeptide according to the present invention.

The composition, food, biological sample protectant and cosmetic according to the present invention are characterized in comprising the above antifreeze protein according to the present invention and/or the above polypeptide according to the present invention.

The method for inhibiting freezing according to the present invention is characterized in comprising the step of adding the above antifreeze protein according to the present invention and/or the above polypeptide according to the present invention to a solution.

The above antifreeze protein according to the present invention and/or the above polypeptide according to the present invention is used for inhibiting freezing.

MODES FOR CARRYING OUT THE INVENTION

The first antifreeze protein (which does not form part of the present invention) is characterized in being derived from a plant, and comprising at least one of the following proteins:

(1) a protein having the amino acid sequence of SEQ ID NO: 1 and having a molecular weight of 19, 000 ± 200 Da as measured by SDS-PAGE;
(2) a protein having the amino acid sequence of SEQ ID NO: 2 and having a molecular weight of 22, 000 ± 250 Da as measured by SDS-PAGE;
(3) a protein having a molecular weight of 59,000 ± 600 Da as measured by SDS-PAGE and having a modified N-terminal;
(4) a protein having the amino acid sequence of any one of the proteins (1) to (3) with one or more amino acid deletions, substitutions or additions, and having an antifreezing activity.

In the present invention, the antifreeze protein widely refers to a protein having a function inhibiting growth of ice crystal. The antifreeze protein which shows an antifreezing activity demonstrated by any one of known methods such as observation of the structure of ice crystal and measurement of ice-crystal growth inhibiting characteristic is included in the range of the present invention.

Hereinafter, properties of the first antifreeze protein are described in detail. The antifreeze protein contains at least one of proteins having a molecular weight of 19,000 $\pm$ 200 Da, 22,000 $\pm$ 250 Da or 59,000 $\pm$ 600 Da as measured by SDS-PAGE.

The protein having a molecular weight of 19,000 $\pm$ 200 Da has an amino acid sequence which is substantially homologous to the amino acid sequence of SEQ ID NO: 1. The protein having a molecular weight of 22,000 $\pm$ 250 Da has an amino acid sequence which is substantially homologous to the amino acid sequence of SEQ ID NO: 2. The protein having a molecular weight of 59,000 $\pm$ 600 Da is subjected to any N-terminal modification. In the present invention, the phrase "an amino acid sequence substantially homologous" refers to overlapping at least not less than 80%, more preferably not less than 90%, further preferably not less than 95%, further preferably not less than 98%, and most preferably 100% of the amino acid sequence.

In addition, the first antifreeze protein may be a protein which contains an amino acid sequence having deletion, substitution or addition of one or a plurality of amino acids relative to the amino acid sequence of any of the above-described proteins and which has an antifreezing activity. In the above amino acid sequences, the number of the amino acid to be deleted, substituted or added is more preferably not less than 1 and not more than 3, further preferably 1 or 2, and particularly preferably 1.

As long as the above amino acid sequences are contained, the protein may be a monomer consisting of a single subunit or a complex including a plurality of subunits, or may contain a part of such a complex. In the present invention, a subunit is, for example, recognized as a low molecular separate band when the protein is analyzed by SDS-PAGE in the presence of a reducing agent such as dithiothreitol, and is a polypeptide which can be obtained by dissociation from the protein.

The phrase "having an antifreezing activity" in the present invention refers to having an activity to inhibit growth and coarsening of ice crystal in the protein solution by inhibiting ice crystallization and controlling the form of ice crystal. More specifically, the antifreeze protein according to the present invention binds to a crystal face of ice crystal to inhibit growth of ice crystal. At which time, the form of ice crystal may change as compared with the case without the antifreeze protein according to the present invention. In addition, further binding of free water to the ice crystal is blocked by the binding of the antifreeze protein, so that ice crystallization is inhibited.

The first antifreeze protein can be obtained in an adsorbed fraction of a chromatography using an anion-exchange resin under a condition of pH 8.0. In addition, the first antifreeze protein preferably precipitates at an acetone concentration of not more than 30% by volume. In the present invention, an anion-exchanger is not particularly limited, and is exemplified by DEAE (Diethylaminoethyl), Q (Quaternary Ammonium) and the like. Also, the upper limit concentration of acetone precipitation is preferably not more than 80% by volume, further preferably not more than 60% by volume, particularly preferably not more than 40% by volume, and most preferably not more than 30% by volume.

The first antifreeze protein can be obtained from a plant. Such a plant is not particularly limited, and is exemplified by a plant belonging to a family Brassicaceae, family Apiaceae, family Liliaceae and family Asteraceae. A plant belonging to family Brassicaceae is exemplified by Chinese cabbage (Brassica rapa L. var. glabra Regel), Japanese radish (Raphanus sativus), broccoli, bok choy (Brassica chinensis L.), komatsuna (Brassica campestris var.peruviridis), turnip (Brassica campestris L.), shirona (Brassica campestris var. amplexicaulis), nozawana (Brassica rapa var. hakabura), hiroshimana (Brassica campeestris), potherb mustard (Brassica rapa var. nipposinica) and mustard (Brassica juncea). A plant belonging to family Apiaceae is exemplified by carrot. A plant belonging to family Liliaceae is exemplified by Welsh onion. A plant belonging to family Asteraceae is exemplified by crown daisy (Chrysanthemum coronarium). An allied species thereof and an improved species thereof may be also used. The antifreeze substance according to the present invention is preferably obtained from, for example, Japanese radish (Raphanus sativus); however, the raw material plant is not particularly limited thereto. Japanese radish (Raphanus sativus) is not particularly limited, and is exemplified by Raphanus sativus var. longipinnatus, Raphanus sativus var. raphanistroides, and Geum japonicum. In addition, when a sprout of the above plants, particularly a Japanese radish sprout, which is referred as Kaiwaredaikon, is used, the first antifreeze protein can be efficiently obtained.

With respect to the term "allied species" in the present invention, for example, an allied species of a family refers to a breed variety which belongs to the same genus but belongs to a genus close to the family to be compared in scientific classification, and an allied species of a specific plant refers to a breed variety which belongs to the same family but is close to the family to be compared in scientific classification. The term "improved species" refers to a plant improved by artificial selection, hybridization, mutation, gene recombination and the like.

The form of a plant is not particularly limited, and may be a seed, a whole plant, and for example, may be a part thereof such as a seed, a sprout, a leaf, and a leaf stem.

The first antifreeze protein may be extracted directly from a plant, and may be extracted after inducing the first antifreeze protein in the plant by a method such as habituation at low temperature.

The temperature for low temperature habituation is not particularly limited, and is preferably not less than 0°C and not more than 20°C. The duration for low temperature habituation is not particularly limited, and habituation for not less than 3 days is preferred.

The first antifreeze protein can be easily extracted, purified and recovered.

The extraction method is not particularly limited, and for example, the first antifreeze protein can be obtained by a known extraction method using water or an organic solvent.

A solvent for extracting the first antifreeze protein is not particularly limited, and one or more solvents selected from the group consisting of water, a hydrophilic organic solvent, supercritical carbon dioxide, subcritical water and the like can be preferably used.

A hydrophilic organic solvent is exemplified by methanol, ethanol and the like. It is preferred that the hydrophilic organic solvent is usable for food processing, and ethanol and the like are exemplified as such a solvent.

Among the solvents, water and ethanol are preferred. Also, it is possible to use a mixed solvent of water and an organic solvent.

When water is used, heated water, particularly hot water, is preferable. When an organic solvent is used, a heated organic solvent is preferable.

The temperature of heated water and a heated organic solvent is not particularly limited. For example, the temperature is preferably not less than 0°C and more preferably not less than 20°C, and preferably not more than 160°C and more preferably not more than 120°C. In addition, an aqueous solvent is exemplified by various buffer solutions such as a sodium acetate buffer solution and a mixed solvent of an alcohol and water; however, an aqueous solvent is not limited thereto. The kind and the amount of extraction solvent can be suitably selected depending on the kind and the amount of a plant subjected to extraction.

The purification method is not particularly limited, and for example, reverse osmosis, ultrafiltration, microfiltration and the like can be suitably used in combination.

The cut-off molecular weight of membrane separation is not particularly limited. When the objective substance is recovered in a fraction which does not permeate a membrane, the lower limit of the cut-off molecular weight is preferably not less than 5,000, more preferably not less than 8,000, even more preferably not less than 10, 000, and most preferably not less than 15, 000. A membrane can be preferably used unless the upper limit thereof exceeds 19,000.

In a membrane separation method, a component having a small molecular weight selectively permeates a membrane. As a result, a component having a large molecular weight in a solution is purified and concentrated. However, permeation performance of a membrane is actually reduced time-dependently due to accumulation of a solute in a solution around the membrane surface (concentration polarization), adsorption of the solute on the membrane surface and in membrane pores, and the like.

When the first antifreeze protein is recovered in a high molecular side, use of a membrane having a cut-off molecular weight of less than 5, 000 is not preferred since removal of a contaminating component in a solution may be insufficient and clogging of a membrane may tend to occur. In addition, a cut-off molecular weight of more than 19,000 substantially makes it difficult to purify and recover the antifreeze protein having a molecular weight of about 19 kDa.

The first antifreeze protein may be further purified as necessary. For example, decantation, filtration, centrifugation and the like may be suitably used in combination to remove a contaminating component. Also, for example, salting out and precipitation by an organic solvent, purification by affinity chromatography, ion exchange column chromatography, gel filtration and the like, as well as concentration by dialysis, ultrafiltration and the like may be suitably carried out in combination.

The second antifreeze protein according to the present invention is characterized by being a plant seed storage protein obtainable from Japanese radish sprout.

The second antifreeze protein according to the present invention includes proteins that show and do not show a thermal hysteresis activity as long as the protein has an antifreezing activity. However, it is preferred that the second antifreeze protein according to the present invention has a very low thermal hysteresis activity in comparison with the antifreezing activity or does not show a thermal hysteresis activity, since the size of ice crystal can be reduced without changing the freezing temperature.

In the present invention, the seed protein refers to a general term for proteins contained in a seed, and a seed storage protein means, for example, a protein which is accumulated in a seed as an energy source necessary for germination of a plant, such as albumin and globulin. Such a seed storage protein can be obtained from a seed of a plant. In addition, the seed storage protein can be obtained also from a sprout, young adult and the like, other than a seed.

The second antifreeze protein according to the present invention is specifically exemplified by being a plant seed storage protein, comprising at least one of the following amino acid sequences (1) to (5) :

(1) an amino acid sequence of SEQ ID NO: 3;
(2) an amino acid sequence of SEQ ID NO: 4;

(3) an amino acid sequence of SEQ ID NO: 5;

(4) an amino acid sequence of SEQ ID NO: 6;

(5) an amino acid sequence corresponding to any one of the amino acid sequence (1) to (4) with not less than 1 and not more than 5 of amino acid deletions, substitutions or additions, and having an antifreezing activity, wherein the antifreeze protein is obtainable from Japanese radish sprout, and wherein said protein is composed of two or more subunits, wherein a molecular weight of at least one subunit is 9000 Da $\pm$ 100 Da or 4000 Da $\pm$ 50 Da as measured by SDS-PAGE.

In the above amino acid sequence (5), the number of the amino acid to be deleted, substituted or added is more preferably not less than 1 and not more than 3, further preferably 1 or 2, and particularly preferably 1.

The meaning of the phrase "having an antifreezing activity" in the present invention is as described above.

As long as the above amino acid sequence is contained, a protein may be a monomer consisting of a single subunit or a complex including a plurality of subunits, or may contain a part of the complex. In the present invention, the subunit is, for example, recognized as a low molecular separate band when the protein is analyzed by SDS-PAGE in the presence of a reducing agent such as dithiothreitol, and is a polypeptide which can be obtained by dissociation from the protein. The part of the complex is exemplified by a subunit having a molecular weight of 9,000 $\pm$ 100 Da and 4,000 $\pm$ 50 Da, and the part of the complex is not particularly limited as long as having an antifreezing activity.

As the second antifreeze protein according to the present invention, the following proteins are preferred:

- the protein which has an antifreezing activity but which has a very low thermal hysteresis activity or no thermal hysteresis activity;
- the protein which is obtained mainly in a non-adsorbed fraction of a chromatography using an anion-exchange resin under a condition of pH 8.0;
- the protein which preferably precipitates at an acetone concentration between not less than 40% by volume and not more than 80% by volume and which can be separated as a precipitate.

The anion-exchange resin is not particularly limited, and is exemplified by DEAE (Diethylaminoethyl), Q (Quaternary Ammonium) and the like. The lower limit concentration of acetone precipitation is preferably not less than 0% by volume, more preferably not less than 20% by volume, and most preferably not less than 40% by volume. The upper limit concentration is preferably not more than 100% by volume, more preferably not more than 80% by volume, and most preferably not more than 60% by volume.

The second antifreeze protein according to the present invention may be directly extracted from a plant and may be extracted after inducing the antifreeze protein in a plant by a method such as habituation at low temperature. A raw material plant is exemplified by plants used in the production of the first antifreeze protein, and when the second antifreeze protein is produced, a seed, a sprout and a young adult of the plant are preferably used.

The temperature for low temperature habituation is not particularly limited, and the lower limit temperature is preferably not less than 0°C, and the upper limit temperature is preferably not more than 20°C. The duration for low temperature habituation is not particularly limited, and habituation for not less than 3 days is preferred.

The second antifreeze protein according to the present invention can be easily extracted, purified and recovered. The specific conditions and the like thereof can be the same as the production conditions of the first antifreeze protein.

However, when the second antifreeze protein according to the present invention is purified by membrane separation, the cut-off molecular weight of membrane separation is not particularly limited. When the objective substance is recovered in a fraction which does not permeate a membrane, the lower limit of the cut-off molecular weight is preferably not less than 250, more preferably not less than 500, further preferably not less than 1,000, and most preferably not less than 2,000, and such a membrane can be preferably used unless the upper limit exceeds 4,000.

When the second antifreeze protein according to the present invention is recovered in a high molecular side, use of a membrane having a cut-off molecular weight of less than 250 is not preferred since removal of a contaminating component in a solution is insufficient and clogging of a membrane tends to occur. In addition, a cut-off molecular weight of more than 4,000 may substantially make it difficult to purify and recover the antifreeze protein containing a small subunit having a molecular weight of about 4 kDa.

The first antifreeze protein (which does not form part of the present invention) and the second antifreeze protein according to the present invention as described above may be optionally solidified into an arbitrary form such as a powder and a granule as necessary. A solidification method is not particularly limited, and is exemplified by a method for powdering the extract according to a conventional means such as spray drying and freeze drying, a method for solidifying the extract to a powdery or granular form by adsorbing or supporting on an excipient, and the like. The above operations are known to a person skilled in the art, and can be appropriately selected depending on the purposes.

The antifreeze protein according to the present invention can be utilized for the purpose of removing impediment caused by crystallization of water in various fields where such impediment is present. For example, the antifreeze protein can be utilized in the fields of foods, machinery, civil engineering, cosmetics, and medicine in which a biological sample is used.

In the field of foods, it is possible to prevent the degradation of taste and others by suppressing crystallization of water contained in a food. For example, it is possible to prevent starch from aging. In addition, when water in a food is crystallized to be an ice, protein, fat component, oil component and the like are physically compressed, and the structure of the components is changed. As a result, taste, quality and the like of a food is deteriorated. When the antifreeze protein is added to a food, such deterioration can be inhibited.

In the fields of machinery and civil engineering, the antifreeze protein according to the present invention can be utilized as a cryoprotective agent for movable part of machinery, road, ground and the like.

In the field of cosmetics, the antifreeze protein according to the present invention can be utilized as an additive for preventing quality degradation of cosmetics. For example, when cosmetics containing an oil component and a fat component are frozen, water contained in the cosmetics may be crystallized to be ice. As a result, the oil component and fat component are physically pressed and the structure thereof is destroyed, whereby the quality and sense of use of the cosmetics become deteriorated. When the antifreeze protein according to the present invention is used, the degradation of quality and the like can be avoided since crystallization of water is prevented and the structure of oil component and fat component is maintained.

In the field of medicine, the antifreeze protein according to the present invention can be utilized as a protectant in cryopreservation of a biological sample. When a biological sample such as a cell, blood and a tissue like an organ is cryopreserved in a conventionally publicly known preservation solution, water in the preservation solution freezes to generate ice crystals. The ice crystal may damage the biological sample. On the other hand, when the antifreeze protein according to the present invention is added thereto, the biological sample can be protected from the damage caused due to ice crystals since generation and growth of ice crystal can be suppressed.

The antifreeze protein of the present invention may have various forms depending on the application thereof. The antifreeze protein may be used as it is, or may be in the form of a solution, a concentrated solution, a suspension, a freeze dried product, a powder, a granule, a tablet and the like.

The antibody used in the present invention reacts specifically with the above-described antifreeze protein and/or the above-described polypeptide. The antibody therefore can be used for confirming the presence or absence of the antifreeze protein and polypeptide in a plant, and specifying the antifreeze protein and polypeptide from a protein mixture.

The antibody used in the present invention may be produced according to a conventional method. For example, a mouse, rat or the like is immunized with the above-described antifreeze protein or polypeptide, and the antibody-producing cell or the splenocyte is fused with a myeloma cell to obtain a hybridoma. The hybridoma is cloned, and a clone producing an antibody which is reactive specifically with the above-described antifreeze protein or polypeptide is screened. The clone is cultured, and a secreted monoclonal antibody may be purified.

Next, the method for measuring the activity of the antifreeze protein according to the present invention and the method for measuring the mass of the protein are described below.

As a method for measuring the antifreezing activity of the antifreeze protein according to the present invention, a method which is appropriately selected depending on the type of a plant and the like. For example, the antifreezing activity can be measured by a known method such as observation of the structure of ice crystal and measurement of an antifreezing activity. When improvement in antifreezing activity is observed with any of methods, the measured protein is included in the scope of the present invention. For example, the antifreezing activity can be measured by cooling a solution of a plant extract containing 30 w/v% sucrose down to -40°C, then raising the temperature up to -6°C , and measuring an average area of ice crystals observed by a microscope. Since the average area of ice crystals is smaller as the antifreezing activity is stronger, the antifreezing activity of a plant extract can be quantitatively evaluated using the value as an index. When the addition of an antifreeze protein leads to any inhibition of formation of ice crystals as compared with a control, the antifreeze protein is considered as having an antifreezing activity.

A method for measuring the mass of a protein in the extract of the present invention is not particularly limited, and the mass can be measured using a known method such as the Lowry method, the bicinchoninic acid (BCA) method and the Bradford method (Coomassie method). The standard protein is not particularly limited, and for example, bovine serum albumin (BSA) can be preferably used.

The antifreeze protein according to the present invention can be easily obtained from a Japanese radish sprout that is a food. Therefore, the antifreeze protein according to the present invention has a very high safety for a living body. Also, the antifreeze protein according to the present invention is contained in a seed of a plant, a sprout and the like in a large amount, thus can be provided in a large amount at low cost. Furthermore, the antifreeze protein of the present invention can be added to a food to help quality maintenance of a frozen food and the like. In addition, the antifreeze protein of the present invention and a composition containing the protein can also be effectively used as a biological sample protectant for freeze preservation of a biological sample such as an organ, a cell, whole blood, a blood component such as a platelet. The protein can also be used as cosmetics having a protective effect of the skin, and the like.

EXAMPLES

Hereinafter, the embodiment of the present invention is described in more detail with Examples. The present invention is not limited to the following Examples in any way, and some of the details can be variously changed. In addition, the present invention is not limited to the above-described embodiments, and various changes may be made within the scope of the claims. An embodiment obtained by a proper combination of disclosed technical means is also included in the technical scope of the present invention.

Production Example 1-1: Extraction

In 60 ml of Tris-HCl (5 mM, pH 8.0), 300 mg of dried powder of Japanese radish sprout extract was dissolved to give a crude extract solution.

Production Example 1-2: Centrifugation

A 300 ml-volume beaker was charged with 60 ml of the crude extract solution obtained in Production Example 1-1, and heated using a water bus (manufactured by EYELA) at 50°C for 30 minutes. Then, the crude extract solution was centrifuged at 10,000 × g for 15 minutes. The supernatant after centrifugation was recovered.

Production Example 1-3: Acetone Fractionation

To 56 ml of the supernatant after centrifugation obtained in Production Example 1-2, acetone cooled down to -30°C was added dropwise little by little. Addition of acetone was continued until the final concentration became 30% by volume. The mixture was centrifuged at 10,000 × g for 15 minutes, and the precipitate was recovered. The resulting precipitate was lyophilized, and then dissolved in 6 ml of Tris-HCl (5 mM, pH 8.0).

Production Example 1-4: Ion Exchange Column Chromatography

The concentrated solution obtained in Production Example 1-3 was diluted with 50 ml of a Tris-HCl buffer (10 mM, pH 8.0). A DEAE column (1.6 × 10 cm, manufactured by GE Healthcare) was equilibrated with the same buffer solution, and charged with 50 ml of the solution. Then, the column was eluted with NaCl gradient from 0 to 0.5 M at a flow rate of 5 ml/min, to recover an adsorbed fraction.

Production Example 1-5: Gel Filtration Column Chromatography

The solvent of the DEAE-non-adsorbed active fraction (52 ml) obtained in Production Example 1-4 was replaced with 2 ml of Tris-HCl (5 mM, pH 8.0). The obtained solution was charged into a gel filtration column (Superdex 200, manufactured by GE Healthcare), and the column was eluted at a flow rate of 1. 3 ml/min. As a result, a protein with a peak observed around 25 kDa was obtained.

Test Example 1-1: Measurement of Protein Concentration and Antifreezing Activity

For each of the solutions obtained in Production Examples 1-1 to 1-4, the protein concentration and the antifreezing activity were measured.

(1) Measurement of Protein Concentration

The protein concentration was measured by the BCA method.

(2) Measurement of Antifreezing Activity

Sucrose was added to each of the solutions obtained in Production Examples 1-1 to 1-4 at a rate of 30 w/v%. Under a microscope having a stage with cooling control function, the solution was cooled down to -40°C, and then the temperature was raised up to -6°C. In the state of keeping -6°C, the average area of the ice crystals observed with a microscope for 30 minutes was measured. As a control, the same measurement was carried out for a 30 w/v% sucrose solution. The result is shown in Table 1. The value in the table shows a relative area when the ice crystal area of the control is 1.0, and a smaller average area of ice crystals shows stronger antifreezing activity.

Table 1

| | Production Example 1-1 | Production Example 1-2 | Production Example 1-3 | Production Example 1-4 | Control |
|---|---|---|---|---|---|
| Average area of ice crystals | 0.35 | 0.53 | 0.25 | 0.21 | 1.0 |
| Protein concentration (mg/ml) | 6.0 | 6.0 | 1.1 | 1.3 | - |

It is clear from Table 1 that the activity per protein of Production Example 1-4 increased more than the activity of Production Example 1-1, and the antifreeze protein was concentrated.

Test Example 1-2: Measurement of Molecular Weight by SDS-PAGE

The active fraction obtained by DEAE column chromatography in Production Example 1-4 was electrophoresed using an SDS-polyacrylamide gel (12.5% gel, manufactured by ATTO) at 20 mA for 90 minutes. The gel after electrophoresis was stained by silver to visualize the bands of proteins. From the result of the gel staining, it was confirmed that the active fraction contained proteins of about 19 kDa, about 22 kDa and about 59 kDa.

Test Example 1-3: Determination of Amino Acid Sequence

The solution (20 μl) containing 50 μg of the purified Japanese radish sprout antifreeze protein obtained in Test Example 1-2 and 20 μl of a sample buffer (manufactured by ATTO, EzApply) were mixed in a volume ratio of 1:1, and the mixture was heated at 99°C for 3 minutes. The sample was applied on a 15% polyacrylamide gel (manufactured by ATTO, e-Page1), and SDS-PAGE was performed at 20 mA for 90 minutes. The gel after SDS-PAGE was transcribed on a PVDF membrane (manufactured by Millipore Corporation, Immobilon PSQ) by the semi-dry method, and CBB staining was performed. The stained spots were cut out, and the N-terminal amino acid sequence was determined by the Edman method using protein sequencer PPSQ-33A manufactured by Shimadzu Corporation. The resulting sequences were as described below.
SEQ ID NO:1 (about 19 kDa): Gly Phe Glu Ser Thr Lys Cys Met Cys Thr
SEQ ID NO:2 (about 22 kDa): Met Ala Lys Glu Ala Gln Lys Cys Gln Cys

On the other hand, the sequence of about 59 kDa protein could not be determined. It was considered that the reason is because the N-terminus of the protein was subjected to some sort of modification.

Production Example 2-1: Extraction of Antifreeze Protein from Japanese Radish Sprout

Commercially available Japanese radish sprouts (200 g) was subjected to extraction with water at 50°C for 2 hours, and the extract was lyophilized to obtain a dried powder. The obtained extract (220 mg) was dissolved in 40 ml of Tris-HCl (5 mM, pH 8.0), to give a crude solution.

Production Example 2-2: Heat Treatment

The crude extract solution obtained in Production Example 2-1 (40 ml) was heated using a constant temperature tank (manufactured by EYELA) at 50°C for 30 minutes, then centrifuged at 10,000 × g for 15 minutes. The supernatant after centrifugation was recovered.

Production Example 2-3: Acetone Fractionation

To 31 ml of the supernatant after centrifugation obtained in Production Example 2-2, acetone cooled down to -30°C was added dropwise little by little. Addition of acetone was continued until the final concentration became 40% by volume. The mixture was centrifuged at 10,000 × g for 15 minutes, and the supernatant was recovered. To the supernatant, acetone cooled down to -30°C was further added dropwise. Addition of acetone was continued until the final concentration became 80% by volume. The mixture was centrifuged at 10,000 × g for 15 minutes, and the precipitate was recovered. The obtained precipitate was dried, and then dissolved in 12 ml of Tris-HCl (5 mM, pH 8.0).

Production Example 2-4: Ion Exchange Column Chromatography

The solution obtained in Production Example 2-3 was diluted with Tris-HCl (5 mM, pH 8.0) to make the total volume to be 50 ml, and charged into a DEAE column (1.6 × 10 cm, manufactured by GE Healthcare) which was equilibrated with the same buffer solution. The same buffer solution was flown at a flow rate of 5 ml/min, and the non-adsorbed fraction was eluted to be recovered.

Production Example 2-5: Gel Filtration Column Chromatography

The solvent of the DEAE-non-adsorbed active fraction obtained in Production Example 2-4 (52 ml) was replaced with 2 ml of Tris-HCl (5 mM, pH 8.0). The solution was charged into a gel filtration column (Superdex 200, manufactured by GE Healthcare), and the column was eluted at a flow rate of 1.3 ml/min. As a result, a protein with a peak observed around 25 kDa was obtained.

Test Example 2-1: Measurement of Protein Concentration, Antifreezing Activity and Thermal Hysteresis Activity

For each of the solutions obtained in Production Examples 2-1 to 2-5, the protein concentration, the antifreezing activity and the thermal hysteresis activity were measured.

(1) Measurement of Protein Concentration

The protein concentration was measured by the BCA method.

(2) Measurement of Antifreezing activity

Sucrose was added to each of the solutions obtained in Production Examples 2-1 to 2-5 at a rate of 30 w/v%. Under a microscope having a stage with cooling control function, the solution was cooled down to -40°C, and then the temperature was raised up to -6°C. In the state of keeping -6°C, the average area of the ice crystals observed with a microscope for 30 minutes was measured. As a control, the same measurement was carried out for a 30 w/v% sucrose solution. The result is shown in Table 2. The value in the table shows a relative area when the ice crystal area of the control is 1.0, and a smaller average area of ice crystals shows stronger antifreezing activity.

(3) Measurement of Thermal Hysteresis Activity

A phase-contrast microscope which was capable of low temperature control was used for the measurement. A glass petri dish was maintained at -20°C, and 1 $\mu$l of the sample was put thereon. The temperature was cooled down to -40°C at a rate of 100°C/min to form ice crystals. The formed ice crystals were warmed up to -5°C at a rate of 100°C/min, and the ice crystals were melted at a rate of 5°C/min, to form single crystals. Next, the temperature was decreased at a rate of 1°C/min and the time point that the single crystal ice crystals started to grow was measured to calculate thermal hysteresis by the following formula.

$$\text{Thermal Hysteresis (°C)} = 60 - 1(\text{°C/sec}) \times \text{Measured Time (sec)}$$

Table 2

|  | Production Example 2-1 | Production Example 2-2 | Production Example 2-3 | Production Example 2-4 | Production Example 2-5 | Control |
|---|---|---|---|---|---|---|
| Average area of ice crystals | 0.37 | 0.49 | 0.32 | 0.69 | 0.46 | 1.0 |
| Protein concentration (mg/ml) | 5.4 | 5.3 | 1.9 | 0.72 | 1.0 | - |

It is clear from Table 1 that the activity per protein concentration was gradually increased and the antifreeze protein was purified while the activity was somewhat decreased after heat treatment in Production Example 2-2. Thermal hysteresis could not be confirmed in any samples of Production Examples 2-1 to 2-5.

Test Example 2-2: Determination of Molecular Weight

The solution (10 μl) containing 5 μg of the purified Japanese radish sprout antifreeze protein of Production Example 2-5 was mixed with 10 μl of a sample buffer (manufactured by ATTO, EzApply) in a volume ratio of 1:1, and the mixture was heated at 99°C for 3 minutes. The obtained sample was applied on a 15% polyacrylamide gel (manufactured by ATTO, e-Pagel), and SDS-PAGE was performed at 20 mA for 85 minutes. The gel after electrophoresis was stained by silver to visualize bands of proteins. From the result of the gel staining, it was confirmed that the solution contained proteins of about 9 kDa and about 4 kDa. In addition, the active fraction obtained by gel filtration chromatography in Production Example 5 had a molecular weight of about 25 kDa. The chromatography chart in Production Example 2-5 had one peak, showing that a single protein was obtained. Therefore, it is clear that a complex was formed, and it was considered that the antifreeze protein contained subunits having a molecular weight of 9 kDa and 4 kDa at a rate of 2 : 2.

Test Example 2-3: Determination of Amino Acid Sequence

The solution (10 μl) containing 50 μg of the purified Japanese radish sprout antifreeze protein of Production Example 2-5 and 10 μl of a sample buffer (manufactured by ATTO, EzApply) were mixed in a volume ratio of 1 : 1, and the mixture was heated at 99°C for 3 minutes. The sample was applied on a 15% polyacrylamide gel (manufactured by ATTO, e-PAGEL), and SDS-PAGE was performed at 20 mA for 85 minutes. The gel after SDS-PAGE was transcribed on a PVDF membrane (manufactured by Millipore Corporation, Immobilon PSQ) by the semi-dry method, and CBB staining was performed. The stained spots were cut out, and the N-terminal amino acid sequence was determined by the Edman method using protein sequencer PPSQ-33A manufactured by Shimadzu Corporation. The resulting sequences are as described below.
SEQ ID NOs: 3 and 4 (about 9 kDa): Pro Gln Gly Pro Gln Gln Arg Pro Pro Leu Leu Gln Gln Cys Cys Asn Glu Leu Xa Gln (Xa is Pro or His)
SEQ ID NOs: 5 and 6 (about 4 kDa): Pro Ala Gly Pro Phe Arg Ile Pro Arg Xb Arg Lys Glu Phe Gln Gln Ala Xc His Leu Arg Ala Cys Gln Gln (Xb is Cys or Asn, and Xc is Gln or Glu)

The PTH amino acids corresponding to 14th and 15th amino acids in SEQ ID NOs: 3 and 4 and the 23rd amino acid in SEQ ID NOs: 5 and 6 were not detected; but the amino acids were presumed as Cys. The sequences showed high homology to large subunit and small subunit of napin that is a known seed storage protein.

Production Example 2-6: Extraction of Antifreeze Protein from Japanese Radish Seed

Commercially available Japanese radish sprout seeds (manufactured by TOHOKU SEED Co., LTD.) (3 g) were pulverized and charged into a 100 ml-volume beaker, and 50 ml of deionized water was added thereto. The mixture was subjected to extraction treatment in a hot water bath at 55°C for 2 hours, and a solid content was removed by filtration. The activity of the obtained filtrate was measured by the method of Test Example 2-1. The result is shown in Table 3. The value in the table shows a relative area when the ice crystal area of the control is 1.0. In addition, SDS-PAGE was performed by the method of Test Example 2-2.

Table 3

|  | Production Example 2-6 | Control |
|---|---|---|
| Average area of ice crystals | 0.48 | 1.0 |
| Protein concentration (mg/ml) | 1.0 | - |

It was confirmed from Table 3 that a Japanese radish seed extract clearly made the size of ice crystals to be small as compared to the control, and had an antifreezing activity. In addition, the band of the seed storage protein as same as in Test Example 2-2 was confirmed by SDS-PAGE of the extract.

Test Example 2-4: Effect of Adding to Meat 1

Chilled chicken leg was cut into 2 cm lengths, and the Japanese radish sprout extract solution obtained in Production Example 2-1 containing 1% salt (diluted so as to be 100 mg/1 kg of meat) was added thereto in an amount of 20% mass of the raw material, then the mixture was tumbled. The mixture was frozen by individually quick-frozen (IQF) method, then allowed to stand still at 4°C for 24 hours to be thawed. The amount of drip after thawing was measured. As a control, a solution containing only 1% salt was used. The result is shown in Table 4.

Table 4

| Solution to be added | Amount of drip |
|---|---|
| 1% salt + Japanese radish sprout extract (100mg/1kg of meat) | 2.9% |
| 1% salt: control | 5.0% |

As shown in Table 4 , it is clear that when Japanese radish sprout extract solution was added, the amount of drip was decreased and quality after thawing was improved.
In addition, each chicken leg after thawing was cooked and eaten by five subjects, and the texture was evaluated by the following 5-levels.

Evaluation Standard

As compared to control chicken,

1: hard, 2: somewhat hard, 3: no difference, 4: somewhat soft, 5: soft

As a result, all subjects evaluated the texture as "soft" or "somewhat soft". It was considered that the result was obtained by inhibiting the growth of ice crystals and protecting cells using the antifreeze protein according to the present invention.

Test Example 2-5: Effect of Adding to Meat 2

Chilled pork boston butt was diced into 1.5 cm pieces, and the Japanese radish sprout extract solution obtained in Production Example 2-1 containing 1% salt (diluted so as to be 1,000 mg or 100 mg/1 kg of meat) was added thereto in an amount of 20% by weight of the raw material, then the mixture was tumbled. The mixture was frozen by individually quick-frozen (IQF) method, then allowed to stand still at 4°C for 24 hours to be thawed. The amount of drip after thawing was measured. As a control, a solution containing only 1% salt was used. The result is shown in Table 5.

Table 5

| Solution to be added | Amount of drip |
|---|---|
| 1% salt + Japanese radish sprout extract (1000mg/1kg of meat) | 5.6% |
| 1% salt + Japanese radish sprout extract (100mg/1kg of meat) | 5.8% |
| 1% salt: control | 5.8% |

As shown in Table 5, there was no difference in the amount of drip. However, when texture was evaluated as same as in Test Example 2-4, all subjects evaluated the texture as "soft" or "somewhat soft". It was considered that the result was obtained by inhibiting the growth of ice crystals and protecting cells using the antifreeze protein according to the present invention.

<110> KANEKA CORPORATION

<120> ICE CRYSTALLIZATION-SUPPRESSING PROTEIN

<130> F11-019PCT

<150> JP2010-105846

<151> 2010-04-30

<150> JP2010-105847
<151> 2010-04-30

<160> 6

<210> SEQ ID NO: 1
<211> 10
<212> PRT
<213> Raphanus sativus

<400> SEQ ID NO: 1

```
                    Gly Phe Glu Ser Thr Lys Cys Met Cys Thr
                                     5                  10
```

<210> SEQ ID NO: 2
<211> 10
<212> PRT
<213> Raphanus sativus

<400> SEQ ID NO: 2

```
                    Met Ala Lys Glu Ala Gln Lys Cys Gln Cys
                                     5                  10
```

<210> SEQ ID NO: 3
<211> 20
<212> PRT
<213> Raphanus sativus

<400> SEQ ID NO: 3

```
            Pro Gln Gly Pro Gln Gln Arg Pro Pro Leu Leu Gln Gln Cys Cys
                             5                  10                  15
            Asn Glu Leu Pro Gln
                             20
```

<210> SEQ ID NO: 4
<211> 20
<212> PRT
<213> Raphanus sativus

<400> SEQ ID NO: 4

```
            Pro Gln Gly Pro Gln Gln Arg Pro Pro Leu Leu Gln Gln Cys Cys
                             5                  10                  15
            Asn Glu Leu His Gln
                             20
```

<210> SEQ ID NO: 5
<211> 25
<212> PRT
<213> Raphanus sativus

<400> SEQ ID NO: 5

```
        Pro Ala Gly Pro Phe Arg Ile Pro Arg Cys Arg Lys Glu Phe Gln
                          5                   10                  15
        Gln Ala Glx His Leu Arg Ala Cys Gln Gln
                         20                   25
```

<210> SEQ ID NO: 6
<211> 25
<212> PRT
<213> Raphanus sativus

<400> SEQ ID NO: 6

```
        Pro Ala Gly Pro Phe Arg Ile Pro Arg Asn Arg Lys Glu Phe Gln
                          5                   10                  15
        Gln Ala Glx His Leu Arg Ala Cys Gln Gln
                         20                   25
```

**Claims**

1. An antifreeze protein, being a plant seed storage protein, comprising at least one of the following amino acid sequences (1) to (5) :

   (1) an amino acid sequence of SEQ ID NO:3;
   (2) an amino acid sequence of SEQ ID NO:4;
   (3) an amino acid sequence of SEQ ID NO:5;
   (4) an amino acid sequence of SEQ ID NO:6;
   (5) an amino acid sequence corresponding to any one of the amino acid sequence (1) to (4) with not less than 1 and not more than 5 of amino acid deletions, substitutions or additions, and having an antifreezing activity,

   wherein the antifreeze protein is obtainable from Japanese radish sprout,
   and
   wherein said protein is composed of two or more subunits,
   wherein a molecular weight of at least one subunit is 9000 Da $\pm$ 100 Da or 4000 Da $\pm$ 50 Da as measured by SDS-PAGE.

2. The antifreeze protein according to claim 1, wherein the antifreeze protein is not adsorbed on an anion-exchange column at pH 8.

3. The antifreeze protein according to claim 2, wherein the anion-exchange column is a DEAE column.

4. The antifreeze protein according to any one of claims 1 to 3, contained in a fraction precipitated under an acetone concentration of not less than 40 vol% and not more than 80 vol% in an acetone fractionation.

5. A polypeptide, obtained by dissociating the antifreeze protein according to any one of claims 1 to 4, and having an antifreezing activity.

6. A composition, comprising the antifreeze protein according to any one of claims 1 to 4 and/or the polypeptide according to claim 5.

7. A food, comprising the antifreeze protein according to any one of claims 1 to 4 and/or the polypeptide according to claim 5.

8. The antifreeze protein according to any one of claims 1 to 4 and/or the polypeptide according to claim 5 for use in inhibiting freezing.

**Patentansprüche**

1. Gefrierschutzprotein, welches ein Speicherprotein aus einem Pflanzensamen ist, umfassend wenigstens eine der folgenden Aminosäuresequenzen (1) bis (5):

 (1) eine Aminosäuresequenz von SEQ ID NO: 3;
 (2) eine Aminosäuresequenz von SEQ ID NO: 4;
 (3) eine Aminosäuresequenz von SEQ ID NO: 5;
 (4) eine Aminosäuresequenz von SEQ ID NO: 6;
 (5) eine Aminosäuresequenz, welche einer der Aminosäuresequenzen (1) bis (4) mit nicht weniger als 1 und nicht mehr als 5 Aminosäuredeletionen, - substitutionen oder -additionen entspricht und welche Gefrierschutzaktivität aufweist,

 wobei das Gefrierschutzprotein aus einem Spross des Japanischen Rettichs erhältlich ist, und
 wobei das Protein aus zwei oder mehr Untereinheiten aufgebaut ist,
 wobei ein Molekulargewicht wenigstens einer Untereinheit gemessen durch SDS-PAGE 9000 Da $\pm$ 100 Da oder 4000 Da $\pm$ 50 Da ist.

2. Gefrierschutzprotein gemäß Anspruch 1, wobei das Gefrierschutzprotein nicht an eine Anionenaustauschsäule bei pH 8 adsorbiert wird.

3. Gefrierschutzprotein gemäß Anspruch 2, wobei die Anionenaustauschsäule eine DEAE-Säule ist.

4. Gefrierschutzprotein gemäß einem der Ansprüche 1 bis 3, enthalten in einer Fraktion, die bei einer Acetonkonzentration von nicht weniger als 40 Vol-% und nicht mehr als 80 Vol-% in einer Aceton-Fraktionierung präzipitiert wird.

5. Polypeptid, welches durch das Dissoziieren des Gefrierschutzproteins gemäß einem der Ansprüche 1 bis 4 erhalten wird und Gefrierschutzaktivität aufweist.

6. Zusammensetzung, welche das Gefrierschutzprotein gemäß einem der Ansprüche 1 bis 4 und/oder das Polypeptid gemäß Anspruch 5 umfasst.

7. Nahrungsmittel, welches das Gefrierschutzprotein gemäß einem der Ansprüche 1 bis 4 und/oder das Polypeptid gemäß Anspruch 5 umfasst.

8. Gefrierschutzprotein gemäß einem der Ansprüche 1 bis 4 und/oder Polypeptid gemäß Anspruch 5 zur Verwendung bei der Verhinderung von Gefrieren.

**Revendications**

1. Protéine antigel, étant une protéine de stockage de graine végétale, comprenant au moins une des séquences d'acides aminés (1) à (5) suivantes :

 (1) une séquence d'acides aminés de SEQ ID NO : 3 ;
 (2) une séquence d'acides aminés de SEQ ID NO : 4 ;
 (3) une séquence d'acides aminés de SEQ ID NO : 5 ;
 (4) une séquence d'acides aminés de SEQ ID NO : 6 ;
 (5) une séquence d'acides aminés correspondant à l'une quelconque des séquences d'acides aminés (1) à (4) avec pas moins de 1 et pas plus de 5 délétions, substitutions ou additions d'acide aminé, et ayant une activité antigel,

 dans laquelle la protéine antigel peut être obtenue à partir d'une pousse de radis japonais,
 et
 dans laquelle ladite protéine est composée de deux sous-unités ou plus,
 dans laquelle un poids moléculaire d'au moins une sous-unité est 9000 Da $\pm$ 100 Da ou 4000 Da $\pm$ 50 Da tel que mesuré par SDS-PAGE.

**2.** Protéine antigel selon la revendication 1, dans laquelle la protéine antigel n'est pas adsorbée sur une colonne échangeuse d'anions à pH 8.

**3.** Protéine antigel selon la revendication 2, dans laquelle la colonne échangeuse d'anions est une colonne de DEAE.

**4.** Protéine antigel selon l'une quelconque des revendications 1 à 3, contenue dans une fraction précipitée sous une concentration en acétone de pas moins de 40 % en volume et pas plus de 80 % en volume dans un fractionnement d'acétone.

**5.** Polypeptide, obtenu en dissociant la protéine antigel selon l'une quelconque des revendications 1 à 4, et ayant une activité antigel.

**6.** Composition, comprenant la protéine antigel selon l'une quelconque des revendications 1 à 4 et/ou le polypeptide selon la revendication 5.

**7.** Aliment, comprenant la protéine antigel selon l'une quelconque des revendications 1 à 4 et/ou le polypeptide selon la revendication 5.

**8.** Protéine antigel selon l'une quelconque des revendications 1 à 4 et/ou polypeptide selon la revendication 5 pour une utilisation dans l'inhibition du gel.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004024237 A
- JP 2004275008 A
- JP 2007153834 A
- JP 2010105846 A
- JP 2010105847 A

### Non-patent literature cited in the description

- *Biophysics,* 2003, vol. 43 (3), 130-135
- *Plant Physiology,* 1999, vol. 119, 1361-1369
- *Biochem. J.,* 1999, vol. 340, 385-391
- *Can. J. Microbiol.,* 1998, vol. 144, 6